# EUROPEAN PATENT SPECIFICATION

(11) **EP 3 078 274 B1**
(45) Date of publication and mention of the grant of the patent: **02.03.2022**
(21) Application number: 16172211.1
(22) Date of filing: 29.04.2005
(51) Int. Cl.: A23K 20/20, A23K 50/10, A61K 33/04, A61K 33/24, A61K 33/26, A61K 33/30, A61K 33/32, A61K 33/34, A61P 3/02, C05D 9/02, C05G 5/23

(54) **TRACE ELEMENTS**
SPURENELEMENTE
OLIGO-ÉLÉMENTS

(30) Priority: 03.05.2004 ZA 200403320
(43) Date of publication of application: 12.10.2016
(62) Divisional of application: 05731763.8
(73) Proprietor: Warburton Technology Limited, Dublin 2 (IE)
(72) Inventor: LAURIE, Robert Naylor, 7130 Somerset West (ZA); SMITH, William Alfred, Dublin, 2 (IE)
(74) Representative: Tetzner, Michael

(56) References cited:
- WO-A1-83/01559
- AU-A- 3 505 899
- DE-A1- 4 128 760
- GB-A- 2 022 998
- US-B2- 6 638 539

## Description

The present invention relates to trace elements.

### BACKGROUND TO INVENTION

Most feeds and feeding systems for farm animals around the world are subject to a sub-optimal status or even a deficiency of essential trace elements. There is a need to optimise trace mineral status in animals in order to optimise production and reproduction efficiency. Various strategies and products have been developed to provide the required trace elements to such animals. Different chemical compounds and complexes have been investigated for supplementing the trace elements by way of licks, drenches or injections.

In general the problem with injectable solutions is that concentration of the minerals in the solutions is too low. This results in relatively large quantities having to be injected, which in turn causes tissue damage and the danger of abscesses at the site of injection. Furthermore, it is seldom the case that individual trace elements are deficient. Normally two or more trace elements are concurrently deficient. Most injectable trace element supplements contain only one trace element and this could result in multiple injections.

ZA 1982/6778 (Laurie) discloses a trace element solution and a method of providing the trace elements to livestock. These trace element solution include ethylene diamino tetra acetic acid complex of the required mineral in suitable quantities. However, the trace element solution includes no selenium or selenite compound.

In the description, the specification and claims hereinafter the expression EDTA refers to ethylene diaminotetraacetic acid (C₁₀H₁₆O₈N₂ or (HO₂CH₂C)₂NCH₂CH₂N-(CH₂CO2H)₂).

US 4,335,116 (Howard) discloses mineral-containing therapeutic compositions containing EDTA complexes of trace elements. Notably, US 4,335,116 utilises tetra-sodium EDTA, a selenium glycine complex, and metal chlorides for the preparation of the EDTA complexes. This method has the following drawbacks:
(a) The chloride ions cause contamination;
(b) Each complex solution is to be made individually;
(c) Overnight time is required for complexing;
(d) Heating up afterward to speed up the process, requires extra apparatus;
(e) If mixtures are required, the individual solutions are blended;
(f) If various concentrations as well as compositions are to be made, it can only be done in a cumbersome way, requiring extra apparatus;
(g) A further problem arises when mixtures of high concentration are needed. In certain cases it would be impossible to deliver them, because mixing is always accompanied by dilution.

US 6,638,539 (Laurie et al) discloses a method of preparing a trace element solution, which includes the steps of providing at least one EDTA-complex, of providing a sodium selenite solution, and of combining the EDTA-complexes and the sodium selenite solution. However, the method enables production of a trace element solution of only about 55 mg/ml.

It is an object of the invention to suggest methods and means for overcoming these problems.

GB 2 022 998 A discloses pharmaceutical compositions comprising water soluble compounds of B, F, Mg, V, Mn, Fe, Co, Ni, Cu, Zn and Mb, glycine, glycerine, ascorbic acid, a salt of a 2,4,5,7- tetrahalofluorescein, a salt of EDTA, and (vii) potassium sodium tartrate. AU 1999 35058 A1 discloses a liquid, multicomponent concentrate of complex plant nutrients and biogenic elements, comprising 10 g to 45 g of iron and 4 g to 30 g of zinc and/or manganese and/or copper and/or boron, and further 5 mg to 350 mg of selenium in 1 liter, and in which the complexing agent for the complex compounds is citric acid and/or ammonium and/or alkali salts thereof. DE 4 128 760 A1 discloses Foodstuffs containing trace elements complexed with biologically usable organic molecules.

### SUMMARY OF INVENTION

The present invention provides an injectable trace element solution for livestock according to claim 1.

### Description of Embodiments

According to the invention, the trace element solution comprises 35-50 mg/ml of zinc, 2.5-10 mg/ml of selenium, 5-20 mg/ml of copper, 7.5-15 mg/ml of manganese and comprises a concentration of the metals of at least 60 mg/ml.

Further, the trace element solution may comprise

Yet further, the trace element solution may comprise

At least one of the metals selected from the group comprising copper, zinc, and manganese is provided in the form of an EDTA complex.

At least one of the metals selected from the group comprising iron and chromium may be provided in the form of an EDTA complex.

The solution may include selenium in the form of a selenite and/or a selenate. The solution may include iodine.

The iodine may be provided in the form of potassium iodide and/or sodium iodide. The concentration of iodine may be between 20 to 400 mg/ml.

The EDTA complex may be obtained by means of at least one compound selected from the group comprising sodium EDTA and potassium EDTA.

The solution may include chloro-cresol as preservative.

The solution may be prepared in a continuous batch process.

The solution is an injectable solution.

In an embodiment not according to the claimed invention, the solution may be a drenchable solution.

In an embodiment not according to the claimed invention, the solution may be provided in the form of a stock-lick.

In an embodiment not according to the claimed invention, a trace element solution is described, which includes at least one compound selected from the group comprising iodine, potassium iodine and sodium iodine and which includes a concentration of the compound(s) of at least 20 mg/ml.

The solution may include chromium.

The chromium may be provided in the form of CrCl₃.6H₂O.

In an embodiment not according to the claimed invention, a method of preparing a trace element solution is described, which includes at least two metals selected from the group comprising selenium, copper, zinc, manganese, iron and chromium and which comprises a concentration of the metals of at least 60 mg/ml, said method including the steps of
(a) providing at least one metal salt of at least one metal selected from the group comprising copper, zinc, manganese, iron and chromium in a container; and
(b) providing in the container at least one compound selected from the group comprising potassium EDTA, sodium EDTA and a sodium hydroxide EDTA mixture to obtain the trace element solution

The method may include the step of adding selenium to the container.

The selenium may be provided in the form of selenite and/or selenate.

The method may include the step of adding CrCl₃.6H₂O to the trace element solution.

The method may include the step of adjusting the pH of the trace element solution to 6,7 to 7,0.

The method may include the step of adjusting the pH of the trace element solution by adding at least one compound selected from the group comprising NaOH and EDTA.

The method may include the step of adding water to the container.

The initial temperature of the solution in which the metal salt(s) are to be chelated may be at least 60 degrees Celsius.

The method may include the step of adding water having a temperature of at least 70 degrees Celsius to the container.

At least one of the metal salt(s) may be selected from the group comprising ZnO, CuCO₃, Na₂CO₃, MnSO₄, FeCl₃ and MnCO₃.

The addition of the EDTA/NaOH mixture may occur gradually with small quantities.

The method may include the step of cooling the trace element solution prior to addition of the selenium.

The MnCO₃ mixture may be prepared by mixing MnSO₄ and Na₂CO₃.

In an embodiment not according to the claimed invention, a stock lick is described, which includes a trace element solution as prepared by a method as described herein.

In an embodiment not according to the claimed invention, a method of providing trace elements to animals such as livestock is described, which includes the steps of preparing a trace element solution as described herein, and of providing the solution in a suitable quantity to an animal.

### DESCRIPTION OF EXAMPLES

The invention will now be described by way of example of injectable solutions in accordance with the invention.

### EXAMPLE 1

Example 1 relates to a method to prepare a trace element solution predominantly to be used for cattle and includes the mineral elements Selenium, Copper and Chromium.

The method enables preparation of 25 litres of the solution containing 40 mg Zn, 10 mg Mn, 5 mg Se, 15 mg Cu and 5 mg Cr per ml.

### A. Preparing MnCO₃

In a suitable container/drum, the MnCO₃ mud is prepared by adding solutions of 900 g MnSO₄ and 1150 g Na₂CO₃ together. The resultant mixture is decanted and washed three times.

### B. Continuous Batch Process

To the MnCO₃ mud, hot water (70°C) is added to a volume of at least 15 litres. Critical is the temperature at the start of the batch process which should be at least 60°C.

### B.1 Preparing MnEDTA

2000 g EDTA and 500 g NaOH are weighed; the EDTA and NaOH are mixed; the EDTA/NaOH mixture is added to the drum, in small quantities to prevent excessive frothing, until the reaction is complete (leaving a clear pinkish solution).

### B.2 Preparing ZnEDTA (2 steps)

### Step 1:

2600 g EDTA, 690 g NaOH and 700 g ZnO are weighed, the EDTA and NaOH are mixed and ZnO is kept separate. The EDTA/NaOH mixture is added to the drum in small quantities to prevent boiling over, followed by addition of the ZnO. The reaction is allowed to complete (again leaving a clear pink solution). The temperature at this stage could reach 103°C.

### Step 2:

2600 g EDTA, 690 g NaOH and 700 g ZnO are weighed. The EDTA and NaOH are mixed and the ZnO kept separate. The EDTA/NaOH mixture is added to the drum in small quantities to prevent boiling over, where after the ZnO is added. The reaction is allowed to complete (again leaving a clear pink solution). The temperature at this stage could reach 103°C.

### B.3 Preparing CuEDTA

1760 g EDTA, 462 g NaOH and 693 g basic CuCO₃ are weighed. The EDTA and NaOH are mixed and the CuCO₃ kept separate. The EDTA/NaOH mixture is added to the drum, followed by careful addition of the CuCO₃, to prevent excessive frothing, and the reaction is allowed to complete (leaving a clear blue solution).

### B.4 25 g chlorocresol is added and stirred till dissolved.

### B.5 23 litres is made up

### B.6 The mixture is allowed to cool to room temperature.

### C. Final phase

### C.1 303 g Na₂SeO₃ is added.

### C.2 The pH is adjusted to 6,7 by adding NaOH (40% solution) or EDTA.

### C.3 738 g EDTA, 192 g NaOH and 641 g CrCl₃.6H₂O are weighed. The EDTA and NaOH are mixed and added to the drum. The CrCl₃.6H₂O is added, whereby the reaction is slow.

### C.4 The volume is made up to 25 litres.

### EXAMPLE 2

Example 2 relates to a method to prepare a trace element solution predominantly to be used for sheep and includes the mineral elements Selenium and Copper.

The method enables preparation of 100 litres of the solution containing 40 mg Zn, 10 mg Mn, 3 mg Se and 10 mg Cu per ml.

### A. Preparing MnCO₃

In a suitable container/drum, the MnCO₃ mud is prepared by adding solutions of 3600 g MnSO₄ and 4600 g Na₂CO₃ together. The mixture is decanted and wash three times.

### B. Continuous Batch Process

To the MnCO₃ mud, is added hot water (70°C) to a volume of at least 60 litres. The temperature at the start of the batch process is critical and should be at least 60°C.

### B.1 Preparing MnEDTA

8000 g EDTA and 2000 g NaOH are weighed. The EDTA and NaOH are mixed. The EDTA/NaOH mixture is added to the drum, in small quantities to prevent excessive frothing, until the reaction is complete (leaving a clear pinkish solution).

### B.2 Preparing ZnEDTA (2 steps)

### Step 1:

10400 g EDTA, 2760 g NaOH and 2800 g ZnO are weighed. The EDTA and NaOH are mixed and the ZnO kept separate. The EDTA/NaOH mixture is added to the drum in small quantities to prevent boiling over, followed by addition of the ZnO. The reaction is allowed to complete (again leaving a clear pink solution). The temperature at this stage could reach 103°C.

### Step 2:

10400 g EDTA, 2760 g NaOH and 2800 g ZnO are weighed. The EDTA and NaOH are mixed and the ZnO kept separate. The EDTA/NaOH mixture is added to the drum in small quantities to prevent boiling over, followed by addition of the ZnO. The reaction is allowed to complete (again leaving a clear pink solution). The temperature at this stage could reach 103°C.

### B.3 Preparing CuEDTA

4646 g EDTA, 1220 g NaOH and 1835 g basic CuCO₃ are weighed. The EDTA and NaOH are mixed and the CuCO₃ kept separate. The EDTA/NaOH mixture is added to the drum, followed by careful addition of the CuCO₃, to prevent excessive frothing, and the reaction is allowed to complete (leaving a clear blue solution).

### B.4 100 g chlorocresol is added and the mixture stirred until dissolved.

### B.5 The volume is made up to 96 litres

### B.6 The mixture is cooled to room temperature.

### C. Final phase

### C.1 728 g Na₂SeO₃ is added.

### C.2 The pH is adjusted to 6,7 by adding NaOH (40% solution) or EDTA.

### C.3 The volume is made up to 100 litres.

### EXAMPLE 3

Example 3 relates to a method to prepare a trace element solution predominantly to be used for cattle and includes the mineral elements Selenium and Copper.

The method enables preparation of 100 litres of the solution containing 40 mg Zn, 10 mg Mn, 5 mg Se and 15 mg Cu per ml.

### A. Preparing MnCO₃

In a suitable container/drum, the MnCO₃ mud is prepared by adding solutions of 3600 g MnSO₄ and 4600 g Na₂CO₃ together. The mixture is decanted and wash three times.

### B. Continuous Batch Process

To the MnCO₃ mud, hot water (70°C) is added to a volume of at least 60 litres. The temperature at the start of the batch process is critical and should be at least 60°C.

### B.1 Preparing MnEDTA

7840 g EDTA and 1960 g NaOH are weighed. The EDTA and NaOH are weighed. The EDTA/NaOH mixture is added to the drum, in small quantities to prevent excessive frothing, until the reaction is complete (leaving a clear pinkish solution).

### B.2 Preparing ZnEDTA (2 steps)

### Step 1:

10400 g EDTA, 2760 g NaOH and 2800 g ZnO are weighed. The EDTA and NaOH are mixed and the ZnO kept separate. The EDTA/NaOH mixture is added to the drum, in small quantities to prevent boiling over, followed by addition of the ZnO. The reaction is allowed to complete (again leaving a clear pink solution). The temperature at this stage could reach 103°C.

### Step 2:

10400 g EDTA, 2760 g NaOH and 2800 g ZnO are weighed. The EDTA and NaOH are mixed and the ZnO kept separate. The EDTA/NaOH mixture is added to the drum, in small quantities to prevent boiling over, followed by addition of the ZnO. The reaction is allowed to complete (again leaving a clear pink solution). The temperature at this stage could reach 103°C.

### B.3 Preparing CuEDTA

7040 g EDTA, 1848 g NaOH and 2780 g basic CuCO₃ are weighed. The EDTA and NaOH are mixed and the CuCO₃ kept separate. The EDTA/NaOH mixture is added to the drum, followed by careful addition of the CuCO₃, to prevent excessive frothing, and the reaction is allowed to complete (leaving a clear blue solution).

### B.4 100 g chlorocresol is added and the mixture stirred till dissolved.

### B.5 The mixture is made up to 96 litres

### B.6 The mixture is allowed to cool to room temperature.

### C. Final phase

### C.1 1212 g Na₂SeO₃ is added.

### C.2 The pH is adjusted to 7,0 by adding NaOH (40% solution) or EDTA.

### C.3 The volume is made up to 100 litres.

### GENERAL

The invention therefore provides a trace element solution which is tissue friendly, i.e. is not damaging or irritant to the tissue of animals and which comprises selenium, copper, zin cand manganese and at a concentration of the metals of at least 60 mg/ml. The trace elements in solution are in a scientifically formulated ratio according to the post-absorption requirements of the animals calculated according to provided As an example the trace element solution comprises
(a) 35-50 mg/ml of zinc;
(b) 10-15 mg/ml manganese;
(c) 5-10 mg/ml selenium;
(d) 5-20 mg/ml copper;
(e) 5-10 mg/ml chromium;
(f) 5-50 mg/ml iron; and
(g) 20-400 mg/ml iodine.

The iodine is provided in the form of potassium iodide or sodium iodide and the iron is provided in the form of iron chloride.

The method of preparing a trace element solution thus enables the production of a solution comprising an adequate trace mineral concentration so that a 5 to 10 milliliter subcutaneous injection can make a significant impact on the trace mineral status of the animal, i.e. a practically applicable injectable supplement and a product that can improve the trace mineral status of an animal is provided. This is important as livestock producers will only inject livestock if a real benefit can be demonstrated. Furthermore, the subcutaneous injection is the preferred route to minimize tissue damage.

## Claims

1. An injectable trace element solution for livestock, which includes 35-50 mg/ml of zinc, 2.5-10 mg/ml selenium, 5-20 mg/ml of copper and 7.5-15 mg/ml of manganese, in which at least one of the metals selected from the group comprising copper, zinc, and manganese is provided in the form of an EDTA complex, and which comprises a concentration of the metals of at least 60 mg/ml.

2. A solution as claimed in claim 1, which includes selenium in the form of a selenite and/or a selenate.

3. A solution as claimed in any one of the preceding claims, which includes iodine.

4. A solution as claimed in claim 3, in which the iodine is provided in the form of potassium iodide and/or sodium iodide.

5. A solution as claimed in claim 3 or claim 4, in which the concentration of iodine is between 20 to 400 mg/ml.

6. A trace element solution according to claim 1, which comprises 5-10 mg/ml chromium.

7. A trace element solution according to claim 1, which comprises 5-50 mg/ml iron.

## Patentansprüche

1. Injizierbare Spurenelementlösung für Vieh, welche 35-50 mg/ml Zink, 2,5-10 mg/ml Selen, 5-20 mg/ml Kupfer und 7.5-15 mg/ml Mangan enthält, wobei wenigstens eines der Metalle ausgewählt aus der Gruppe, die Kupfer, Zink und Mangan umfasst, in der Form eines EDTA-Komplexes bereitgestellt ist, und welche eine Konzentration der Metalle von 60 mg/ml umfasst.

2. Lösung nach Anspruch 1, welche Selen in der Form eines Selenits oder eines Selenats enthält.

3. Lösung nach einem der vorherigen Ansprüche, welche Iod enthält.

4. Lösung nach Anspruch 3, wobei das Iod in der Form von Kaliumiodid und/oder Natriumiodid bereitgestellt ist.

5. Lösung nach Anspruch 3 oder 4, wobei die Konzentration von Iod zwischen 20 und 400 mg/ml beträgt.

6. Lösung nach Anspruch 1, welche 5-10 mg/ml Chrom umfasst.

7. Lösung nach Anspruch 1, welche 5-50 mg/ml Eisen umfasst.

## Revendications

1. Solution injectable d'oligoéléments destinée au bétail, solution qui contient de 35 à 50 mg/ml de zinc, de 2,5 à 10 mg/ml de sélénium, de 5 à 20 mg/ml de cuivre et de 7,5 à 15 mg/ml de manganèse, solution dans laquelle au moins l'un des métaux sélectionnés parmi le groupe se composant de cuivre, de zinc et de manganèse est fourni sous la forme d'un complexe EDTA, et solution dont la concentration des métaux est au moins 60 mg/ml.

2. Solution selon la revendication 1, qui contient du sélénium sous la forme d'un sélénite et/ou d'un séléniate.

3. Solution selon l'une des revendications précédentes, qui contient de l'iode.

4. Solution selon la revendication 3, dans laquelle l'iode est fourni sous la forme d'iodure de potassium et/ou d'iodure de sodium.

5. Solution selon la revendication 3 ou la revendication 4, dans laquelle la concentration en iode est comprise entre 20 et 400 mg/ml.

6. Solution d'oligoéléments selon la revendication 1, qui contient de 5 à 10 mg/ml de chrome.

7. Solution d'oligoéléments selon la revendication 1, qui contient de 5 à 50 mg/ml de fer.
